(19) Europäisches Patentamt
European Patent Office
Office européen des brevets

(11) Publication number: **0 323 885**
**A1**

# (12) EUROPEAN PATENT APPLICATION

(21) Application number: **89300011.7**

(22) Date of filing: **04.01.89**

(51) Int. Cl.[4]: **C 07 D 257/04**

(30) Priority: **05.01.88 GB 8800099**

(43) Date of publication of application:
**12.07.89 Bulletin 89/28**

(84) Designated Contracting States:
**AT BE CH DE ES FR GB GR IT LI LU NL SE**

(71) Applicant: **MAY & BAKER LIMITED**
**Dagenham Essex RM10 7XS (GB)**

(72) Inventor: **Griffiths, Nigel David**
**c/o May & Baker Limited Dagenham**
**Essex RM10 7XS (GB)**

**Hibberd, Brynley James W.F.**
**c/o May & Baker Limited Dagenham**
**Essex RM10 7XS (GB)**

**Thatcher, Alan Arthur**
**c/o May & Baker Limited Dagenham**
**Essex RM10 7XS (GB)**

(74) Representative: **Bentham, Stephen et al**
**J.A. Kemp & Co. 14 South Square Gray's Inn**
**London WC1R 5EU (GB)**

## (54) New chemical process.

(57) A process for the preparation of a di(alkali metal) salt of tetrazole-5-carboxylic acid comprises the reaction, in an anhydrous tertiary amine base medium containing a strong acid, of an alkali metal azide with an alkyl cyanoformate followed, without isolation of the alkyl tetrazole-5-carboxylate thus produced, by reaction in situ with an alkali metal hydroxide: the alkali metal salt of tetrazole-5-carboxylic acid obtained can be used to prepare pharmaceuticals.

EP 0 323 885 A1

**Description**

## NEW CHEMICAL PROCESS

THIS INVENTION relates to a new process for the preparation of salts of tetrazole-5-carboxylic acid, and their use in the preparation of pharmaceuticals.

In British Patent Specification No. 2006782B and in the specifications of corresponding patents and patent applications filed outside Great Britain, for example United States Patent No. 4442115, there is described inter alia a process for the preparation of compounds of the general formula shown in Figure I of the drawings assembled at the end of the present specification [wherein $R^1$ represents a straight- or branched-chain alkylsulphonyl or alkylsulphamoyl group, each such group containing from 1 to 6 carbon atoms, a dialkylsulphamoyl or dialkylcarbamoyl group (wherein the two alkyl groups may be the same or different and each contains from 1 to 4 carbon atoms), a straight- or branched-chain alkanoyl, alkoxycarbonyl or alkylcarbamoyl group containing from 2 to 6 carbon atoms, a cycloalkylcarbonyl group containing from 3 to 8 carbon atoms in the cycloalkyl moiety, or a formyl, trifluoroacetyl, sulphamoyl, cyano, carbamoyl, aralkanoyl (e.g. phenylacetyl), or aroyl (e.g. benzoyl) group, $R^2$ represents a halogen atom or a straight- or branched-chain alkyl, alkoxy, alkylthio, alkylsulphonyl, or alkylsulphamoyl group, each such group containing from 1 to 6 carbon atoms, a dialkylsulphamoyl, dialkylamino or dialkylcarbamoyl group (wherein the two alkyl groups may be the same or different and each contains from 1 to 4 carbon atoms), a straight- or branched-chain alkanoyl, alkoxycarbonyl, alkoxycarbonylamino, alkylcarbamoyl or alkanoylamino group containing from 2 to 6 carbon atoms, a cycloalkylcarbonyl group containing from 3 to 8 carbon atoms in the cycloalkyl moiety, or a formyl, nitro, trifluoromethyl, trifluoroacetyl, aryl (e.g. phenyl), benzyloxycarbonylamino, sulphamoyl, cyano, tetrazol-5-yl, carbamoyl, benzyloxy, aralkanoyl (e.g. phenylacetyl), or aroyl (e.g. benzoyl) group, or a group of the formula:

$-CR^3=NOR^4$ II

(wherein $R^3$ represents a hydrogen atom or a straight- or branched-chain alkyl group containing from 1 to 5 carbon atoms, an aryl (e.g. phenyl), aralkyl (e.g. benzyl) or trifluoromethyl group, or a cycloalkyl group containing from 3 to 8 carbon atoms, and $R^4$ represents a hydrogen atom, or a straight- or branched-chain alkyl group containing from 1 to 6 carbon atoms optionally substituted by a phenyl group, or represents an aryl (e.g. phenyl) group optionally substituted by one or more substituents selected from halogen atoms and straight- or branched-chain alkyl and alkoxy groups containing from 1 to 6 carbon atoms and hydroxy, trifluoromethyl and nitro groups), and n represents zero or an integer 1 or 2, the substituents $R^2$ being the same or different when n represents 2] by the reaction of a compound of the general formula shown in Figure III of the drawings (wherein $R^1$, $R^2$ and n are as hereinbefore defined) with the dipotassium salt of tetrazole-5-carboxylic acid, in the presence of N,N-dimethyl(chloromethyleneimmonium) chloride or a source thereof (for example a mixture of dimethylformamide and an acid chloride such as oxalyl chloride), preferably near or below room temperature, e.g. between -25° and +35°C.

The compounds of the formula shown in Figure I possess valuable pharmacological properties, in particular properties of value in the treatment of allergic conditions, for example respiratory disorders such as those manifested by the interaction of tissue-fixed antibodies with specific antigens, such as allergic bronchial asthma.

In the aforementioned specifications the preparation of the dipotassium salt of tetrazole-5-carboxylic acid is described as follows, using ethyl cyanoformate and an azide, such as sodium azide, trimethylsilyl azide or tributyltin azide, as starting materials, isolating ethyl tetrazole-5-carboxylate as an intermediate, and formation of the dipotassium salt therefrom after reaction with potassium hydroxide.

Ethyl tetrazole-5-carboxylate was prepared by the following methods:-

(a) Ethyl cyanoformate (2.5g) was dissolved in dry pyridine (10 ml) and the stirred solution was treated with a cooled mixture of trifluoroacetic acid (4.4 ml) and dry pyridine (15 ml). The stirred suspension was treated with sodium azide (1.8 g) and the resulting mixture was stirred at 60°-65°C for 48 hours. The mixture was cooled to room temperature and poured onto a mixture of ice and concentrated hydrochloric acid (100 g), and extracted with diethyl ether (3 x 30 ml) and the combined ethereal extracts were dried over magnesium sulphate and evaporated to dryness to give an oil [which partially crystallised on trituration with petroleum ether (b.p. 40°-60°C)] which was purified by chromatography on silica gel, eluting with a mixture (1:1 v/v) of petroleum ether (b.p. 40°-60°C) and diethyl ether, to give ethyl tetrazole-5-carboxylate, m.p. 88°-93°C, (1.57 g; yield = 45% based upon ethyl chloroformate).

(b) By proceeding in a similar manner, but replacing the pyridine by the appropriate quantity of 2,6-dimethylpyridine, there was obtained ethyl tetrazole-5-carboxylate, m.p. 80°-92°C (yield = 35% based upon ethyl chloroformate).

(c) A solution of trimethylsilylazide (0.58g) and ethyl cyanoformate (0.5g) in dry benzene (20ml) was heated at reflux for 48 hours. The solution was allowed to stand at room temperature for 4 days, and the resulting white solid was filtered off to give ethyl tetrazole-5-carboxylate, m.p. 88°-93°C (0.3 g; yield = 37% based upon ethyl chloroformate).

(d) By proceeding in a similar manner, but replacing the benzene by the appropriate quantity of ethyl acetate, there was obtained ethyl tetrazole-5-carboxylate, m.p. 86°-90°C (0.5 g; yield = 61% based upon ethyl chloroformate).

(e) A solution of tributyltin azide (6.65 g) and ethyl cyanoformate (1.98 g) in dry benzene (25 ml) was heated at reflux for 48 hours. The benzene was removed under reduced pressure and the residue was dissolved in an excess quantity of a dry solution of hydrogen chloride in ethanol. The mixture was allowed to stand, and then the ethanol was removed in vacuo on the rotary evaporator to give a red oil.

Chromatography of this oil on a column of silica gel, eluting with a mixture of methanol and chloroform (1:9 v/v), gave ethyl tetrazole-5-carboxylate, identical with samples prepared by the other methods as described above.

Dipotassium salt of tetrazole-5-carboxylic acid was prepared as follows:-

Ethyl tetrazole-5-carboxylate (0.36g) was dissolved in hot ethanol (7.5 ml) and the solution was treated with a solution of potassium hydroxide (0.22 g) in water (0.7 ml). The resulting white solid was filtered off, and washed with ethanol to give the dipotassium salt of tetrazole-5-carboxylic acid, which did not melt below 330°C (0.16 g; yield = 33% based upon ethyl tetrazole-5-carboxylate).

In these examples the overall yield of dipotassium salt of tetrazole-5-carboxylic acid based upon ethyl chloroformate is between 33% x 35% = 12% and 33% x 61% = 20%.

It has now been found that important advantages, such as higher yields, easier operational procedures and greater safety, may be obtained by the use of an improved procedure, which involves preparation of the salt in situ, without isolation of the tetrazole-5-carboxylate ester, and this is a feature of the present invention.

As a further feature of the present invention, it has also been found that alternative alkali metal salts of tetrazole-5-carboxylic acid, for example the disodium salt, may be used to replace the dipotassium salt of tetrazole-5-carboxylic acid in the reaction with compounds of the formula shown in Figure III to form compounds of the formula shown in Figure I.

The hitherto unknown alkali metal salts of tetrazole-5-carboxylic acid other than the dipotassium salt constitute a further feature of the present invention.

Another feature of the present invention is constituted by the alkali metal salts of tetrazole-5-carboxylic acid when prepared by the improved process of the present invention.

The present invention accordingly provides a process for the preparation of an alkali metal salt of tetrazole-5-carboxylic acid of the general formula shown in Figure (IV) of the drawings (wherein M represents an alkali metal atom, preferably potassium or sodium) which comprises the reaction, in an anhydrous tertiary amine base medium containing a strong acid, of an alkali metal azide, e.g. sodium azide, with an alkyl cyanoformate, preferably containing from 1 to 4 carbon atoms in the alkyl group, e.g. ethyl cyanoformate, followed without isolation of the alkyl tetrazole-5-carboxylate thus produced, by reaction in situ with an alkali metal hydroxide of the formula:-

MOH (V)

wherein M is as hereinbefore defined, to prepare the alkali metal salt of tetrazole-5-carboxylic acid. The alkyl cyanoformate is generally added to the reaction mixture after the alkali metal azide.

The tertiary amine base is generally an alkylpyridine or dialkylpyridine (the alkyl groups of which preferably contain from 1 to 4 carbon atoms and are preferably methyl) or, preferably pyridine. The strong acid is, for example, p-toluenesulphonic acid or, preferably, trifluoroacetic acid. The reaction of the alkali metal aside and alkyl cyanoformate is usually carried out at temperatures from 15° to 80°C and is preferably followed by treatment with an alkanol (preferably containing from 1 to 4 carbon atoms, e.g. ethanol) and an aqueous solution of the alkali metal hydroxide or with an aqueous-alkanolic solution of the alkali metal hydroxide, at 50° - 80°C, preferably 60 - 65°C; the reaction mixture is preferably cooled to 0°-20°C, and the precipitated salt of the formula shown in Figure IV is then isolated, for example by filtration. The alkali metal ion in the alkali metal azide is preferably the same as the alkali metal in the desired product.

Preferably the strong acid has the following characteristics:- (i) it should be a sufficiently strong acid to liberate hydrazoic acid from the alkali metal azide; and (ii) it should form pyridinium salts (or, as appropriate, alkylpyridinium or dialkylpyridinium salts) which are sufficiently soluble in pyridine (or the alkylpyridine or dialkylpyridine which may be used as an alternative medium). p-Toluenesulphonic acid and, more especially, trifluoroacetic acid, fulfil these requirements.

One or more of the following advantages may be secured by use of the new process more particularly by adopting the preferred process conditions described above:-

1. The yields of the new process are very good or even almost quantitative, and so they are very much higher than the low overall yields of the previously used processes.

2. In the previously used process [variants (a) and (b)] it was necessary to separate ethyl tetrazole-5-carboxylate from trifluoroacetic acid, which was difficult because of the similarity in acid strengths of the two compounds. In the new process the di-alkali metal tetrazole-5-carboxylate products are readily separated from the alkali metal trifluoroacetate or p-toluenesulphonate, which are much more soluble in the reaction mixture and in solvents such as ethanol which can be used to rinse the filtered precipitate of di-alkali metal tetrazole-5-carboxylate. No important quantities of alkali metal trifluoroacetate or p-toluenesulphonate have been found contaminating the product of the new process.

3. In the previously used process [variants (a), (b) and (c)] dangerous, explosive and highly toxic hydrazoic acid gas was liberated in the reaction step of treatment with acid.

On a manufacturing scale this would need to be removed, for example, by passage of nitrogen gas and removal of the gaseous mixture produced.

4. The reaction together of the alkali metal azide and the alkyl cyanoformate in the mixture of pyridine

and acid is strongly exothermic but, in the new process the alkyl cyanoformate is added to the reaction mixture after the alkali metal aside and this facilitates control of the temperature.

5. Exposure of alkyl tetrazole-5-carboxylates to heat has been found, in thermal stability measurements, to be potentially hazardous, with risk of explosion. The new process avoids the possibly dangerous step in the previously used process of evaporation of a solution of an alkyl tetrazole-5-carboxylate in an organic solvent. The di-alkali metal salts have been shown to be thermally stable at up to 300° C (the limit of the test equipment).

6. The new process does not need the expensive steps of chromatography, or extraction with solvents such as diethyl ether.

7. If the alkali metal ion in the alkali metal azide is chosen to be other than the alkali metal M in the desired product, slight contamination of the product, by the salt of the alkali metal from the aside, can occur. This problem can be avoided by using the same alkali metal in the azide and in the hydroxide of formula V. Usually sodium compounds are chosen for economic reasons, for large scale preparations.

The present invention also provides a process for the preparation of compounds of the formula shown in Figure I, especially compounds of the formula shown in Figure I wherein $R^1$ represents a straight- or branched-chain alkanoyl group containing from 2 to 6 carbon atoms, e.g. an acetyl group, and $(R^2)_n$ represents an optional substituent in the position in the ring para with respect to the indicated hydroxy group, for example a halogen atom or a cyano group or a straight- or branched-chain alkyl group containing from 1 to 6, preferably from 1 to 3, carbon atoms, for example
3′-acetyl-2′-hydroxytetrazole-5-carboxanilide,
3′-acetyl-5′-cyano-2′-hydroxytetrazole-5-carboxanilide,
3′-acetyl-2′-hydroxy-5′-methyltetrazole-5-carboxanilide,
3′-acetyl-5′-ethyl-2′-hydroxytetrazole-5-carboxanilide, or, more especially,
3′-acetyl-5′-fluoro-2′-hydroxy-tetrazole-5-carboxanilide,
which process comprises the reaction of an alkali metal salt of tetrazole-5-carboxylic acid prepared by the process of the present invention with a compound of the general formula shown in Figure (III) wherein $R^1$, $R^2$ and n are as hereinbefore defined in the presence of N, N -dimethyl (chloromethyleneimmonium) chloride or a source thereof.

The following Examples illustrate the invention:-

EXAMPLE 1

A stirred slurry of sodium azide (18.0 g) in dry pyridine (200 ml) was treated at 15° C with trifluoroacetic acid (34.7 g). Ethyl cyanoformate (25.0 g) was added and the mixture was then stirred at 65° C for 48 hours. The mixture was then cooled to 60°C and ethanol (150 ml) was added, followed by a solution of potassium hydroxide (35.0 g) in water (35 ml). After stirring at 65° C for 15 minutes the mixture was cooled to room temperature, and filtered, to give dipotassium tetrazole-5-carboxylate (42.5g; yield = 89.4% based upon ethyl cyanoformate) in the form of a white crystalline solid.

EXAMPLE 2

A stirred slurry of sodium azide (62.8 g) in dry pyridine (800 ml) was treated at 15° C with trifluoroacetic acid (121.6 g), followed by ethyl cyanoformate (100 g). The mixture was stirred at 70° - 75° C for 6 hours, and then it was cooled to 60° C and treated with ethanol (600 ml), followed by a solution of sodium hydroxide (88 g) in water (120 ml).

After stirring at 65° C for 15 minutes the mixture was cooled to room temperature and filtered, to give disodium tetrazole-5-carboxylate (157.7 g; yield = 99.8% based upon ethyl cyanoformate), in the form of a white crystalline solid.

EXAMPLE 3

A slurry of disodium tetrazole-5-oarboxylate (90 g) in dimethylformamide (570 ml) at -20° C was treated with thionyl chloride (78 g) during 30 minutes, and the mixture was stirred at -20° C for one hour. A solution of 3-amino-5-fluoro-2-hydroxyacetophenone (96 g) in a mixture of dimethylformamide (240 ml) and pyridine (190 ml) was then added during 30 minutes, maintaining the temperature at -20° C. The mixture was then allowed to warm to room temperature, and then was heated at 90° C for one hour. The resulting mixture was cooled to 20° C, diluted with water (2900 ml), acidified to pH1 by treatment with concentrated hydrochloric acid, and filtered, to give 3′-acetyl-5′-fluoro-2′-hydroxytetrazole-5-carboxanilide, m.p. 230-231° C (with decomposition) (110 g; yield = 72.5%). Recrystallisation from glacial acetic acid gave purified 3′-acetyl-5′-fluoro-2′-hydroxytetrazole-5-carboxanilide, m.p. 232-233° C (95g).

Similar results were obtained by replacing the disodium tetrazole-5-carboxylate, used as starting material, by the appropriate quantity of dipotassium tetrazole-5-carboxylate.

## Claims

1. A process for the preparation of an alkali metal salt of tetrazole-5-carboxylic acid of the general formula:

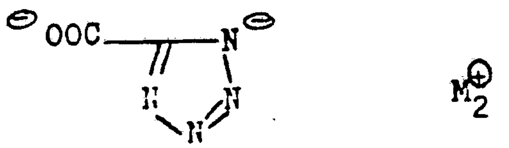

IV

wherein M represents an alkali metal atom which process comprises the reaction, in an anhydrous tertiary amine base medium containing a strong acid, of an alkali metal azide with an alkyl cyanoformate followed, without isolation of the alkyl tetrazole-5-carboxylate thus produced, by reaction in situ with an alkali metal hydroxide of the formula:

MOH (V)

wherein M is as hereinbefore defined to prepare the alkali metal salt of tetrazole-5-carboxylic acid.

2. A process according to claim 1 in which the tertiary amine base is pyridine, an alkylpyridine or a dialkylpyridine.

3. A process according to claim 1 or 2 wherein the strong acid is p-toluenesulphonic acid or trifluoroacetic acid.

4. A process according to any one of the preceding claims in which the alkali metal azide is sodium azide, the alkyl cyanoformate is ethyl cyanoformate, the reaction of the sodium azide and ethyl cyanoformate is carried out at a temperature from 15° to 80° C, and the ethyl tetrazole-5-carboxylate is treated with an alkanol and an aqueous solution of the alkali metal hydroxide or with an aqueous alkanolic solution of an alkali metal hydroxide at a temperature of 50° to 80° C.

5. A process according to any one of the preceding claims followed by the reaction of an alkali metal salt of tetrazole-5-carboxylic acid thus obtained with a compound of the general formula:

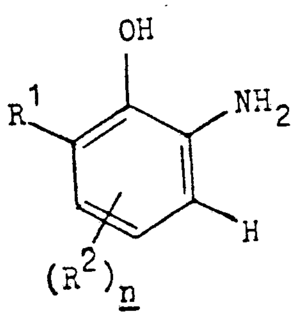

(III)

wherein $R^1$ represents a straight- or branched-chain alkylsulphonyl or alkylsulphamoyl group, each such group containing from 1 to 6 carbon atoms, a dialkylsulphamoyl or dialkylcarbamoyl group (wherein the two alkyl groups may be the same or different and each contains from 1 to 4 carbon atoms), a straight- or branched-chain alkanoyl, alkoxycarbonyl or alkylcarbamoyl group containing from 2 to 6 carbon atoms, a cycloalkylcarbonyl group containing from 3 to 8 carbon atoms in the cycloalkyl moiety, or a formyl, trifluoroacetyl, sulphamoyl, cyano, carbamoyl, aralkanoyl or aroyl group, $R^2$ represents a halogen atom or a straight- or branched-chain alkyl, alkoxy, alkylthio, alkylsulphonyl or alkylsulphamoyl group, each such group containing from 1 to 6 carbon atoms, a dialkylsulphamoyl, dialkylamino or dialkylcarbamoyl group (wherein the two alkyl groups may be the same or different and each contains from 1 to 4 carbon atoms), a straight- or branched-chain alkanoyl, alkoxycarbonyl, alkoxycarbonylamino, alkylcarbamoyl or alkanoylamino group containing from 2 to 6 carbon atoms, a cycloalkylcarbonyl group containing from 3 to 8 carbon atoms in the cycloalkyl moiety, or a formyl, nitro, trifluoromethyl, trifluoroacetyl, aryl, benzyloxycarbonyl amino, sulphamoyl, cyano, tetrazol-5-yl, carbamoyl, benzyloxy, aralkanoyl or aroyl group, or a group of the formula:

$-CR^3=NOR^4$ (II)

(wherein $R^3$ represents a hydrogen atom or a straight- or branched-chain alkyl group containing from 1 to 5 carbon atoms, an aryl, aralkyl or trifluoromethyl group or a cycloalkyl group containing from 3 to 8 carbon atoms, and $R^4$ represents a hydrogen atom or a straight- or branched-chain alkyl group containing from 1 to 6 carbon atoms optionally substituted by a phenyl group or represents an aryl group optionally substituted by one or more substituents selected from halogen atoms and straight- or branched-chain alkyl and alkoxy groups containing from 1 to 6 carbon atoms and hydroxy, trifluoromethyl and nitro groups), and n represents zero or an integer 1 or 2, the substituents $R^2$ being the same or different when n represents 2 in the presence of N,N-dimethyl(chloromethyleneimmonium)chloride or a source thereof, to obtain a compound of the general formula:

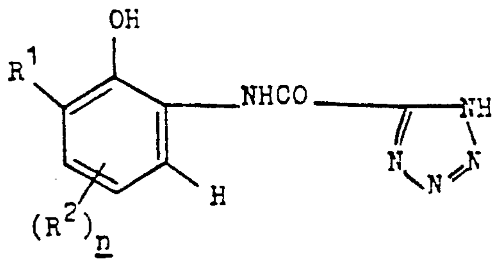

I

wherein $R^1$, $R^2$ and n are as hereinbefore defined.

6. A process according to claim 5 wherein $R^1$ represents a straight- or branched-chain alkanoyl group containing from 2 to 6 carbon atoms and $(R^2)_n$ represents a halogen atom or a cyano group or a straight- or branched-chain alkyl group containing from 1 to 6 carbon atoms in the position in the ring para to the indicated hydroxy group.

7. A process according to claim 5 or 6 for the preparation of a compound of general formula (I) as defined in claim 5 which compound is 3′-acetyl-2′-hydroxytetrazole -5-carboxanilide, 3′-acetyl-5′-cyano-2′-hydroxytetrazole-5-carboxanilide, 3′-acetyl-2′-hydroxy-5′-methyltetrazole-5-carboxanilide or 3′-acetyl-5′-ethyl-2′-hydroxytetrazole-5-carboxanilide.

8. A process according to claim 5 or 6 for the preparation of a compound of general formula (I) as defined in claim 5 which compound is 3′-acetyl-5′-fluoro -2′-hydroxytetrazole-5-carboxanilide.

9. An alkali metal salt of tetrazole-5-carboxylic acid of the general formula (IV) depicted in claim 1 wherein M represents an alkali metal other than potassium.

10. An alkali metal salt according to claim 9 wherein M represents sodium.

European Patent Office

# EUROPEAN SEARCH REPORT

Application Number

EP 89 30 0011

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 316 037 (J.H. SELLSTEDT) * the whole document * --- | 1 | C 07 D 257/04 |
| Y | COMPREHENSIVE HETEROCYCLIC CHEMISTRY vol. 5, part 4A, Oxford 1984, Pergamon Press * page 833, chapter 4.13.5.3 * --- | 1 | |
| X | GB-A-2 006 782 (MAY AND BAKER) * page 6, lines 1-23, page 10, examples 13,14 *; & US -A - 44 42115 (Cat. D) --- | 5 | |
| X | EP-A-0 147 973 (LILLY INDUSTRIES) * page 1, lines 15-21, page 5, lines 1-13 * ----- | 9,10 | |

TECHNICAL FIELDS SEARCHED (Int. Cl.4)

C 07 D 257/00

The present search report has been drawn up for all claims

| Place of search | Date of completion of the search | Examiner |
|---|---|---|
| BERLIN | 21-03-1989 | KYRIAKAKOU G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)